# FASCICULE DE BREVET EUROPEEN

(11) **EP 1 935 398 B1**
(45) Date de publication et mention de la délivrance du brevet: **30.11.2016**
(21) Numéro de dépôt: 07301708.9
(22) Date de dépôt: 19.12.2007
(51) Int. Cl.: A61K 8/58, A61K 8/891, A61K 8/893, A61K 8/894, A61K 8/898, A61Q 5/06

(54) **Utilisation d'une silicone dans une composition de mise en forme des cheveux comprenant un alcoxysilane comprenant un groupe fonctionnel solubilisant.**
Verwendung eines Silikons in einer Zusammensetzung zur Formgebung von Haaren, die ein Alkoxysilan mit einer funktionellen Gruppe enthält, die löslich gemacht werden kann
Use of a silicone in a hair styling compositions including an alkoxysilane containing a functional group for making it soluble

(30) Priorité: 20.12.2006 FR 0655758
(43) Date de publication de la demande: 25.06.2008
(73) Titulaire: L'Oréal, 75008 Paris (FR)
(72) Inventeur: Benabdillah, Katarina, 95130, LE PLESSIS-BOUCHARD (FR); Lerda, Patrice, 75013, PARIS (FR); Rollat-Corvol, Isabelle, 75017, PARIS (FR); Samain, Henri, 91570, BIEVRES (FR)
(74) Mandataire: Caillet, Isabelle

(56) Documents cités:
- EP-A- 1 767 187
- EP-A- 1 767 188
- EP-A- 1 767 189
- EP-A2- 0 159 628
- WO-A-89/04163
- FR-A1- 2 783 164
- FR-A1- 2 783 165
- FR-A1- 2 783 167
- FR-A1- 2 798 063
- US-A- 2 782 790
- US-A- 4 344 763
- STARCH M S: "Silicones in Hair Care Products" DRUG AND COSMETIC INDUSTRY, vol. 134, no. 6, juin 1984 (1984-06), page 38,40,42,44,102, XP002109041
- LARREY ET AL: "HAIR CARE: THE SILICONE SOLUTION. CONTROLLED UPTAKE OF SILICONE ON HAIR" COSMETIC NEWS, MILAN, IT, vol. 21, no. 118, 1998, pages 40-44, XP009082869 ISSN: 1125-6222

## Description

L'invention se rapporte à l'utilisation d'une silicone dans une composition de mise en forme des cheveux comprenant un alcoxysilane à groupe fonctionnel solubilisant, pour éviter la dégradation du toucher des cheveux lors de l'application répétée de ladite composition de mise en forme des cheveux. L'invention se rapporte encore à des compositions cosmétiques particulières contenant une silicone et un alcoxysilane à groupe fonctionnel solubilisant.

Les consommateurs aux cheveux fins et frisés recherchent des effets coiffants durables apportant de la masse, du corps et du volume aux cheveux.

Les produits de coiffage permettent une mise en forme non permanente des cheveux et permettent d'obtenir ces effets coiffants. Ils s'utilisent sur cheveux mouillés ou secs avant la mise en forme à la main ou à l'aide d'une brosse ou d'un peigne. Ils contiennent généralement un ou plusieurs actifs cosmétiques, tels que des polymères fixants, des épaississants, du glycérol, des silicones ou des cires par exemple.

Après leur application sur les cheveux et après séchage, ces produits durcissent de façon importante. Cela se traduit par un toucher corporisé et sec nécessaire au maintien et au volume de la coiffure.

Ces produits de coiffage présentent aussi l'inconvénient que les effets coiffants disparaissent au premier shampooing suivant l'application du produit de coiffage. Il faut donc les appliquer quotidiennement.

Il existe cependant des produits de coiffage qui permettent d'obtenir ces effets coiffants, notamment d'apporter de la masse, du corps et du volume, et qui sont résistants à plusieurs shampooings.

On connaît ainsi des documents FR 2 783 164, FR-2 783 167, FR 2 783 165 et FR 2 798 063 l'utilisation de certains composés organiques du silicium choisis parmi les silanes comprenant un atome de silicium et les siloxanes comprenant deux ou trois atomes de silicium, ces composés étant hydrosolubles, pour le maintien de la coiffure où la mise en forme des cheveux. Ces composés permettent d'obtenir des effets coiffants, par exemple de la masse, du corps, la facilité de démêlage, le dessin de boucle, résistant à plusieurs shampooings. Néanmoins, ces effets sont relativement faibles et irréguliers. Ils s'estompent après environ deux semaines.

On connaît également les documents FR 552 855, FR 552 853 et FR 552 856 des compositions cosmétiques contenant des composés organiques du silicium soluble dans les milieux alcooliques ou hydroalcooliques contenant des fonctions hydrolysables et polymérisables, ces compositions permettant d'obtenir des effets coiffants puissants et résistants à plusieurs shampooings, en général résistants à plusieurs shampooings.

Cependant, après l'application répétée des produits capillaires contenant des composés organiques du silicium, en particulier des alcoxysilanes à groupe fonctionnel solubilisant, les cheveux peuvent devenir secs, rêches, avoir un toucher de cheveux sensibilisés et être abîmés.

Il existe donc un besoin de disposer d'une composition cosmétique de mise en forme des cheveux qui permettent d'obtenir des effets coiffants puissants, résistants à plusieurs shampooings, tout en gardant un toucher de cheveux naturel.

La demanderesse a découvert que l'utilisation d'une silicone dans une composition de mise en forme des cheveux comprenant un alcoxysilane à groupe fonctionnel solubilisant permettait d'éviter la dégradation du toucher des cheveux lors de l'application répétée de ladite composition de mise en forme des cheveux.

L'invention a donc pour objet l'utilisation d'au moins une silicone telle que définie dans la revendication 1 dans une composition cosmétique de mise en forme des cheveux comprenant au moins un alcoxysilane contenant au moins un groupe fonctionnel solubilisant tel que défini dans la revendication 1 pour éviter la dégradation du toucher des cheveux lors de l'application répétée de ladite composition de mise en forme des cheveux.

Par utilisation pour éviter la dégradation du toucher des cheveux, on entend le fait d'éviter que les cheveux deviennent secs, rêches, aient un toucher de cheveux sensibilisés et soient abîmés.

Par groupe fonctionnel solubilisant, on entend au sens de la présente invention un groupe chimique fonctionnel facilitant la mise en solution de l'alcoxysilane dans le solvant ou mélange de solvants de la composition, en particulier dans l'eau ou dans les mélanges hydroalcooliques.

Le groupe fonctionnel solubilisant est, selon la présente invention, choisi parmi les groupes amine primaire, secondaire et tertiaire, amine aromatique, alcool, acide carboxylique, acide sulfonique, anhydride, carbamate, urée, guanidine, aldéhyde, ester, amide, époxy, pyrrole, dihydroimidazole, gluconamide, pyridyle et polyéther.

Le ou les alcoxysilanes présents dans la composition utilisés selon l'invention contiennent un ou plusieurs groupes fonctionnels solubilisants, identiques ou différents, tels que définis précédemment.

Selon l'invention, le ou les alcoxysilane contenant au moins un groupe fonctionnel solubilisant présents dans la composition utilisée selon l'invention sont choisis parmi les composés de formule : 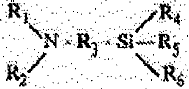 dans laquelle :
R₄ représente un halogène, un groupe OR' ou R'₁ ;
R₅ représente un halogène, un groupe OR" ou R'₂ ;
R₆ représente un halogène, un groupe OR"' ou R'₃ ;
R₁, R₂, R₃, R', R", R"', R'₁, R'₂, R'₃ représentent, indépendamment les uns des autres, un groupe hydrocarboné saturé ou insaturé, linéaire ou ramifié, portant éventuellement des groupes chimiques supplémentaires, R₁, R₂, R', R" et R"' pouvant en outre désigner l'hydrogène, deux au moins des groupes R₄, R₅ et R₆ étant différents des groupes R'₁, R'₂ et R'₃, deux au moins des groupes R', R" et R"' étant différents de l'hydrogène.

De préférence, les groupes R₁, R₂, R', R'₁, R'₂, R'₃, R"et R"' sont choisis parmi les radicaux alkyle de C₁-C₁₂, aryle de C₆ à C₁₄, alkyle de C₁ à C₈-aryle de C₆ à C₁₄, et aryle de C₆ à C₁₄-alkyle de C₁ à C₈.

De préférence, le ou les alcoxysilanes contenant au moins un groupe fonctionnel solubilisant présents dans la composition utilisée selon l'invention sont choisis parmi les composés de formule : 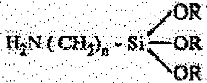 dans laquelle les radicaux R, identiques ou différents, sont choisis parmi les radicaux alkyle en C₁-C₆ et n est un nombre entier de 1 à 6, de préférence de 2 à 4.

De façon particulièrement préférée, l'alcoxysilane contenant au moins un groupe fonctionnel solubilisant présent dans la composition utilisée selon l'invention est le γ-aminopropyl triéthoxysilane.

Le ou les alcoxysilanes contenant au moins un groupe fonctionnel solubilisant représentent généralement entre 0,1 et 20 %, de préférence entre 1 et 15 % en poids du poids total de la composition.

La ou les silicones utilisées selon l'invention sont choisies parmi les polydialkylsiloxanes, notamment les polydiméthylsiloxanes (PDMS), organomodifiés comportant au moins un groupement fonctionnel choisi les groupements aminés, les groupements alcoxy en C₁-C₄, les groupements acides carboxyliques, les groupements acryliques, les groupements polyaminés.

Les silicones utilisables selon l'invention sont des silicones volatiles ou non volatiles, cycliques, linéaires ou ramifiées, modifiées ou non par des groupements organiques, ayant une viscosité de 5.10⁻⁶ à 2,5m²/s à 25°C et de préférence 1.10⁻⁵ à 1 m²/s.

Les silicones utilisables selon l'invention peuvent être solubles ou insolubles dans la composition et en particulier être des polyorganosiloxanes insolubles dans la composition de l'invention. Elles peuvent se présenter sous forme d'huiles, de cires, de résines ou de gommes.

Les organopolysiloxanes sont définis plus en détail dans l'ouvrage de Walter NOLL "Chemistry and Technology of Silicones" (1968), Academie Press. Elles peuvent être volatiles ou non volatiles.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Parmi les silicones organomodifiées, on peut citer les polyorganosiloxanes comportant :
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄. Les silicones aminées peuvent posséder des fonctions alcoxy en C₁-C₄ additionnelles comme celle correspondant au produit WACKER BELSIL ADM LOG 1 ;
- des groupements alcoxylés, comme le produit commercialisé sous la dénomination "SILICONE COPOLYMER F-755" par SWS SILICONES et ABIL WAX® 2428, 2434 et 2440 par la société GOLDSCHMIDT ;
- des groupements anioniques du type acide carboxylique comme, par exemple, dans les produits décrits dans le brevet EP 186 507 de la société CHISSO CORPORATION, ou du type alkyle-carboxylique comme ceux présents dans le produit X-22-3701 E de la société SHIN-ETSU ; 2-hydroxyalkylsulfonate ; 2-hydroxyalkylthiosulfate tels que les produits commercialisés par la société GOLDSCHMIDT sous les dénominations « ABIL® S201 » et « ABIL^{®} S255 » ;
- des groupements acryliques ; on peut citer en particulier les produits commercialisés sous les appellations VS80 et VS70 par la société 3M ;
- des groupements polyaminés.

La ou les silicones utilisées selon l'invention représentent généralement entre 0,01 et 20 %, de préférence entre 0,1 et 10%, mieux entre 0,1 et 5 % en poids du poids total de la composition.

La composition cosmétique utilisée selon l'invention comprend généralement au moins un solvant choisi parmi l'eau, les solvants alcooliques en C₁-C₈, et leurs mélanges.

Les solvants alcooliques en C₁-C₈ sont choisis généralement parmi les alcanols, les alcanediols, l'alcool benzylique et l'alcool phényléthylique.

De préférence, le ou les solvants sont choisis parmi l'éthanol, le propanol et l'isopropanol.

La composition cosmétique utilisée selon l'invention peut comprendre en outre au moins un actif cosmétique additionnel choisi parmi les hydrolysats de protéines, les agents de gonflement et de pénétration, les agents antichute, les agents antipelliculaires, les épaississants naturels ou synthétiques non polymériques, les agents de suspension, les agents séquestrant, les agents réducteurs, les agents opacifiants, les colorants, les filtres solaires, les vitamines ou provitamines, les parfums et les conservateurs, les ajusteurs de pH et leurs mélanges.

Les ajusteurs de pH peuvent être choisis parmi les agents alcalins, tels que, par exemple, l'ammoniaque, la monoéthanolamine, la diéthanolamine, la triéthanolamine, la 1,3-propanediamine, un hydroxyde alcalin, tel que le 2-amino-2-méthyl-1-propanol, ou bien parmi les agents acidifiants tel que par exemple l'acide phosphorique ou l'acide chlorhydrique.

Le pH de la composition utilisée selon l'invention est généralement compris entre 2 et 13, de préférence entre 4 et 11.

Le ou les agents épaississants peuvent être choisis parmi les agents épaississants cellulosiques, par exemple l'hydroxyéthylcellulose, l'hydroxypropylcellulose et le carboxyméthylcellulose, la gomme de guar et ses dérivés, par exemple l'hydroxypropyl guar, commercialisé par la société RHODIA sous la référence JAGUAR HP 105, les gommes d'origine microbienne, telle que la gomme de xanthane et la gomme de scléroglucane, les agents épaississants synthétiques tels que l'alcool cétylstéarylique, les homopolymères réticulés d'acide acrylique ou d'acide acrylamidopropanesulfonique, par exemple le Carbomer, les polymères associatifs non ioniques, anioniques, cationiques ou amphotères, tels que les polymères commercialisés sous les appellations PEMULEN TR1 ou TR2 par la société GOODRICH, SALCARE SC90 par la société ALLIED COLLOIDS, ACULYN 22, 28, 33, 44 ou 46 par la société ROHM & HAAS et ELFACOS T21 0 et T212 par la société AKZO.

A titre d'agents conditionnants utlisables dans la composition cosmétique utilisée selon l'invention, on peut citer le chlorure de béhentrimonium commercialisé par la société CLARIANT sous la référence GENAMIN KDNP.

La composition utilisée selon l'invention peut également contenir un ou plusieurs autres acides organiques.

Les acides organiques additionnels sont généralement choisis parmi les acides comportant une ou plusieurs fonctions acide carboxylique, sulfonique, phosphonique ou phosphorique. Ils peuvent contenir d'autres fonctions chimiques, en particulier des fonctions hydroxy ou amino. Ils peuvent être saturés ou insaturés.

On peut citer en particulier l'acide acétique, l'acide propanoïque, l'acide butanoïque, l'acide lactique, l'acide glycolique, l'acide ascorbique, l'acide maléique, l'acide phtalique, l'acide succinique, la taurine, l'acide tartrique, l'acide gluconique, l'acide glucuronique et l'acide citrique.

La présence d'un ou plusieurs acides organiques dans la composition selon l'invention permet en particulier d'augmenter la proportion d'eau dans la composition.

La composition utilisée selon l'invention peut se présenter sous toutes les formes possibles pour une application sur les cheveux, notamment sous forme d'une solution du type lotion ou sérum, sous forme de gel, sous forme d'émulsion eau-dans-huile, huile-dans-eau ou multiple, de consistance liquide plus ou moins épaisse, telle que des laits et des crèmes plus ou moins onctueuses, ou des mousses.

La composition utilisée selon l'invention peut être une lotion, un gel, une mousse ou une crème de coiffage, une crème de soin, un shampooing, un après-shampooing, ou une composition de coloration.

La composition utilisée selon l'invention peut être conditionnée sous différentes formes : des tubes, des pots mais aussi des vaporisateurs, des flacons pompe ou dans des récipients aérosol, afin d'assurer une application de la composition sous forme vaporisée ou sous forme de mousse.

Lorsque la composition est conditionnée sous forme d'aérosol, l'aérosol peut être bi-compartimenté.

Lorsque la composition est conditionnée dans un dispositif aérosol, elle comprend au moins un agent propulseur, qui peut être choisi parmi les hydrocarbures volatiles, tels que le n-butane, le propane, l'isobutane, le pentane, les hydrocarbures halogénés et leurs mélanges. On peut également utiliser en tant que propulseur le gaz carbonique, le protoxyde d'azote, le diméthyléther (DME), l'azote, ou l'air comprimé. On peut aussi utiliser des mélanges de propulseurs. De préférence, on utilise le diméthyl éther.

Avantageusement, l'agent propulseur est présent à une concentration comprise entre 5 et 90% en poids par rapport au poids total de la composition dans le dispositif aérosol, et, plus particulièrement, à une concentration comprise entre 10 et 60%.

Comme expliqué précédemment, la composition utilisée selon l'invention est une composition de mise en forme des cheveux.

Pour mettre en forme les cheveux, la composition comprenant le ou les alcoxysilanes contenant au moins un groupe fonctionnel solubilisant et la ou les silicones décrite précédemment est appliquée sur les cheveux, rincés ou non. L'application se fait de préférence sous la forme d'un spray, soit à l'aide d'un flacon pompe, soit à l'aide d'un aérosol.

La composition selon l'invention est ensuite laissée à poser sur les cheveux, pour lui permettre de pénétrer dans le cheveu, en général pendant au plus 30 minutes, de préférence pendant 5 à 15 minutes.

Les cheveux peuvent être rincés à l'eau après l'application de la composition et le temps de pose.

Les cheveux peuvent alors être mis en forme, par exemple par brushing, ou par utilisation d'un fer.

De préférence, les cheveux sont séchés au sèche cheveux et mis en forme par brushing.

Lors du séchage des cheveux, le ou les alcoxysilanes contenant au moins un groupe fonctionnel solubilisant, qui sont des composés monomères, sèchent et polymérisent, formant des matériaux insolubles dans l'eau et dans le shampooing.

Les cheveux ayant été mis en forme comme décrit précédemment présentent plus de volume, de masse, de texture, et ils se mettent mieux en forme.

L'effet coiffant obtenu est supérieur à celui obtenu lors de la mise en forme des cheveux sans application de la composition utilisée selon l'invention.

Les cheveux peuvent être lavés par shampooings puis remis en forme par brushing, les même effets coiffants sont obtenus. Les effets sont résistants à plusieurs shampooings.

L'invention a encore pour objet une composition cosmétique comprenant au moins une silicone telle que définie précédemment et au moins un alcoxysilane contenant au moins un groupe fonctionnel solubilisant tel que défini précédemment.

L'invention est illustrée par des exemples suivants.

### Exemples

On formule des compositions cosmétiques de mise en forme des cheveux, destinées à être utilisées selon l'invention.

**Lotion de coiffage 1 :**

| | |
|---|---|
| Aminopropyl triéthoxysilane (Dow Corning) | 10 % m.a. |
| HCl | qs pH=1 0,5 |
| DC 939 Emulsion (Dow Corning) | 1 % m.a. |
| Eau déminéralisée | qsp 100 % |

**Gel de coiffage :**

| | |
|---|---|
| Aminopropyl triéthoxysilane (Dow Corning) | 10 % m.a. |
| HCl | qs pH=10,5 |
| DC 939 Emulsion (Dow Corning) | 1 % m.a. |
| Jaguar HP105 (Rhodia) | 1,5 % m.a. |
| Eau déminéralisée | qsp 100 % |

**Crème de coiffage :**

| | |
|---|---|
| Aminopropyl triéthoxysilane (Dow Corning) | 10 % m.a. |
| HCl | qs pH=1 0,5 |
| DC 939 Emulsion (Dow Corning) | 1 % m.a. |
| Alcool cétylstéarylique | 2,5 % m.a. |
| Genamin KDMP (Clariant) | 0,4 % m.a. |
| Eau déminéralisée | qsp 100 % |

**Lotion de coiffage très traitant pour cheveux frisés :**

| Partie A : | |
|---|---|
| Aminopropyl triéthoxysilane (Dow Corning) | 10 % m.a. |
| HCl | qs pH=1 0,5 |
| Eau déminéralisée | qsp 100% |

| Partie B : | |
|---|---|
| Wacker Belsil ADM LOG1 (Wacker) | 2 % m.a. |
| Eau | qsp 100% |

| | |
|---|---|
| Les parties A et B sont à mélanger avant l'application sur les cheveux. | |

Les compositions sont appliquées sur cheveux humides.

Elles peuvent être laissées à poser 15 minutes, puis les cheveux sont rincés. Les cheveux sont alors séchés à l'air libre, au brushing ou à l'aide de pinces plates.

On peut aussi laisser les cheveux sécher sans rinçage. Les cheveux sont alors laissés séchés librement, au brushing ou à l'aide d'un fer à pinces plates.

On obtient alors des effets coiffant semi-durables, et de bonnes performances cosmétiques, en terme de toucher, de douceur et de souplesse.

Ces bonnes performances sont conservées même après une application répétée de ces compositions.

## Revendications

1. Utilisation d'au moins une silicone dans une composition cosmétique de mise en forme des cheveux comprenant au moins un alcoxysilane contenant au moins un groupe fonctionnel solubilisant choisi parmi les groupes amine primaire, secondaire et tertiaire, amine aromatique, alcool, acide carboxylique, acide sulfonique, anhydride, carbamate, urée, guanidine, aldéhyde, ester, amide, époxy, pyrrole, dihydroimidazole, gluconamide, pyridyle et polyéther, pour éviter la dégradation du toucher des cheveux lors de l'application répétée de ladite composition de mise en forme des cheveux, le ou les alcoxysilanes contenant au moins un groupe fonctionnel solubilisant sont choisis parmi les composés de formule : 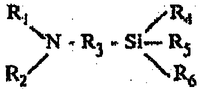 dans laquelle :
R₄ représente un halogène, un groupe OR' ou R'₁ ;
R₅ représente un halogène, un groupe OR" ou R'₂ ;
R₆ représente un halogène, un groupe OR'" ou R'₃ ;
R₁, R₂, R₃, R', R", R"', R'₁, R'₂, R'₃ représentent, indépendamment les uns des autres, un groupe hydrocarboné saturé ou insaturé, linéaire ou ramifié, portant éventuellement des groupes chimiques supplémentaires, R₁, R₂, R', R" et R'" pouvant en outre désigner l'hydrogène, deux au moins des groupes R₄, R₅ et R₆ étant différents des groupes R'₁, R'₂ et R'₃, deux au moins des groupes R', R" et R"' étant différents de l'hydrogène
la ou les silicones étant choisies parmi les polysiloxanes organomodifiés choisis parmi les polydialkylsiloxanes, de préférence, les polydiméthysiloxanes, organomodifiés par des groupements amines, alcoxy en C₁-C₄, carboxyles, acryliques, polyamines.

2. Utilisation selon la revendication 1, **caractérisée en ce que** les groupes R₁, R₂, R', R'₁, R'₂, R'₃, R" et R'" sont choisis parmi les radicaux alkyle de C₁-C₁₂, aryle de C₆ à C₁₄, alkyle de C₁ à C₈-aryle de C₆ à C₁₄, et aryle de Ce à C₁₄-alkyle de C₁ à C₈.

3. Utilisation selon la revendication 1 **caractérisée en ce que** le ou les alcoxysilane contenant au moins un groupe fonctionnel solubilisant sont choisis parmi les composés de formule : 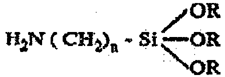 dans laquelle les radicaux R, identiques ou différents, sont choisis parmi les radicaux alkyle en C₁-C₆ et n est un nombre entier de 1 à 6, de préférence de 2 à 4.

4. Utilisation selon la revendication 3, **caractérisée en ce que** l'alcoxysilane contenant au moins un groupe fonctionnel solubilisant est le γ-aminopropyl triéthoxysilane.

5. Utilisation selon l'une quelconque des revendications précédentes **caractérisée en ce que** le ou les alcoxysilanes contenant au moins un groupe fonctionnel solubilisant représentent entre 0,1 et 20 %, de préférence entre 1 et 15 % en poids du poids total de la composition.

6. Utilisation selon l'une quelconque des revendications précédentes **caractérisée en ce que** la ou les silicones représentent entre 0,01 et 20 % en poids, de préférence entre 0,1 et 10 % en poids, mieux entre 0,1 et 5 % en poids du poids total de la composition.

7. Utilisation selon l'une quelconque des revendications précédentes **caractérisée en ce que** la composition comprend au moins un solvant choisi parmi l'eau, les solvants alcooliques en C₁-C₈, et leurs mélanges.

8. Utilisation selon la revendication 7 **caractérisée en ce que** les solvants alcooliques en C₁-C₈ sont choisis parmi les alcanols, les alcanediols, l'alcool benzylique et l'alcool phényl éthylique.

9. Utilisation selon la revendication 8 **caractérisée en ce que** les solvants sont choisis par l'éthanol, le propanol, l'isopropanol.

10. Utilisation selon l'une quelconque des revendications précédentes **caractérisée en ce qu'**elle comprend en outre au moins un actif cosmétique choisi parmi les hydrolysats de protéines, les agents de gonflement et de pénétration, les agents antichute, les agents antipelliculaires, les épaississants naturels ou synthétiques non polymériques, les agents de suspension, les agents séquestrant, les agents réducteurs, les agents opacifiants, les colorants, les filtres solaires, les vitamines ou provitamines, les parfums et les conservateurs, les ajusteurs de pH, les agents conditionnants et leurs mélanges.

11. Utilisation selon l'une quelconque des revendications précédentes **caractérisée en ce qu'**elle comprend un ou plusieurs acides organiques.

12. Utilisation selon la revendication 11 **caractérisée en ce que** le ou les acides organiques sont choisis parmi l'acide acétique, l'acide propanoïque, l'acide butanoïque, l'acide lactique, l'acide glycolique, l'acide ascorbique, l'acide maléique, l'acide phtalique, l'acide succinique, la taurine, l'acide tartrique, l'acide gluconique, l'acide glucuronique et l'acide citrique.

13. Composition cosmétique capillaire comprenant au moins une silicone et au moins un alcoxysilane contenant au moins un groupe fonctionnel solubilisant tel que défini à la revendication 1, la ou les silicones étant choisies parmi les polysiloxanes organomodifiés choisis parmi les polydialkylsiloxanes, de préférence, les polydiméthysiloxanes, organomodifiés par des groupements amines, alcoxy en C₁-C₄, carboxyles, acryliques, polyamines.

## Patentansprüche

1. Verwendung von mindestens einem Silikon in einer kosmetischen Zusammensetzung zur Formgebung von Haaren, die mindestens ein Alkoxysilan umfasst, das mindestens eine löslichkeitserhöhende funktionelle Gruppe enthält, ausgewählt aus der primären, sekundären und tertiären Aminogruppe, der aromatischen Aminogruppe, der Alkohol-, der Carboxylsäure-, der Sulfonsäure-, der Anhydrid-, der Carbamat-, der Harnstoff-, der Guanidin-, der Aldehyd-, der Ester-, der Amid-, der Epoxid-, der Pyrrol-, der Dihydroimidazol-, der Gluconamid-, der Pyridyl- und der Polyethergruppe, um die Verschlechterung des Haargefühls beim wiederholten Aufbringen der Zusammensetzung zur Formgebung von Haaren zu verhindern, wobei das oder die Alkoxysilane, das bzw. die mindestens eine löslichkeitserhöhende funktionelle Gruppe enthält bzw. enthalten, aus Verbindungen mit folgender Formel ausgewählt ist bzw. sind: 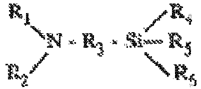 wobei:
R₄ ein Halogen, eine Gruppe OR' oder R'₁ bedeutet;
R₅ ein Halogen, eine Gruppe OR" oder R'₂ bedeutet;
R₆ ein Halogen, eine Gruppe OR"' oder R'₃ bedeutet;
R₁, R₂, R₃, R', R", R"', R'₁, R'₂, R'₃ unabhängig voneinander eine lineare oder verzweigte, gesättigte oder ungesättigte Kohlenwasserstoffgruppe bedeuten, die gegebenenfalls zusätzliche chemische Gruppen trägt, wobei R₁, R₂, R', R" und R"' ferner Wasserstoff bezeichnen können, mindestens zwei der Gruppen R₄, R₅ und R₆ verschieden von den Gruppen R'₁, R'₂ und R'₃ sind, mindestens zwei der Gruppen R', R" und R"' von Wasserstoff verschieden sind, das oder die Silikon(e) aus organomodifizierten Polysiloxanen ausgewählt sind, die aus Polydialkylsiloxanen, vorzugsweise Polydimethylsiloxanen ausgewählt ist bzw. sind, organomodifiziert mit Aminogruppen, C₁-C₄-Alkoxygruppen, Carboxylgruppen, Acrylgruppen, Polyaminogruppen.

2. Verwendung nach Anspruch 1, **dadurch gekennzeichnet, dass** die Gruppen R₁, R₂, R', R'₁, R'₂, R'₃, R" und R"' aus C₁- bis C₁₂-Alkylresten, C₆- bis C₁₄-Arylresten, C₁-C₈-Alkyl-C₆-C₁₄-Arylresten und C₆-C₁₄-Aryl-C₁-C₈-Alkylresten ausgewählt sind.

3. Verwendung nach Anspruch 1, **dadurch gekennzeichnet, dass** das oder die Alkoxysilan(e), das bzw. die mindestens eine löslichkeitserhöhende funktionelle Gruppe enthält bzw. enthalten, aus den Verbindungen mit folgender Formel ausgewählt ist bzw. sind: 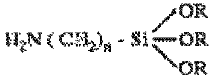 wobei die Reste R, gleich oder verschieden, aus C₁- bis C₆-Alkylresten ausgewählt sind, und n eine ganze Zahl von 1 bis 6, vorzugsweise von 2 bis 4 ist.

4. Verwendung nach Anspruch 3, **dadurch gekennzeichnet, dass** das Alkoxysilan, das mindestens eine löslichkeitserhöhende funktionelle Gruppe enthält, γ-Aminopropyltriethoxysilan ist.

5. Verwendung nach einem der vorhergehenden Ansprüche, **dadurch gekennzeichnet, dass** das oder die Alkoxysilan(e), das bzw. die mindestens eine löslichkeitserhöhende funktionelle Gruppe enthält bzw. enthalten, zwischen 0,1 und 20 Gew.-%, vorzugsweise zwischen 1 und 15 Gew.-% des Gesamtgewichts der Zusammensetzung darstellt bzw. darstellen.

6. Verwendung nach einem der vorhergehenden Ansprüche, **dadurch gekennzeichnet, dass** das oder die Silikon(e) zwischen 0,01 und 20 Gew.-%, vorzugsweise zwischen 0,1 und 10 Gew.-%, besser zwischen 0,1 und 5 Gew.-% des Gesamtgewichts der Zusammensetzung darstellt bzw. darstellen.

7. Verwendung nach einem der vorhergehenden Ansprüche, **dadurch gekennzeichnet, dass** die Zusammensetzung mindestens ein Lösungsmittel umfasst, das aus Wasser, alkoholischen C₁-C₈-Lösungsmitteln und ihren Mischungen ausgewählt ist.

8. Verwendung nach Anspruch 7, **dadurch gekennzeichnet, dass** die alkoholischen C₁-C₈-Lösungsmittel aus Alkanolen, Alkandiolen, Benzylalkohol und Phenylethylalkohol ausgewählt sind.

9. Verwendung nach Anspruch 8, **dadurch gekennzeichnet, dass** die Lösungsmittel aus Ethanol, Propanol, Isopropanol ausgewählt sind.

10. Verwendung nach einem der vorhergehenden Ansprüche, **dadurch gekennzeichnet, dass** sie ferner mindestens einen kosmetischen Wirkstoff umfasst, der ausgewählt ist aus Proteinhydrolysaten, Quell- und Penetriermitteln, Mitteln gegen Haarausfall, Mitteln gegen Schuppen, natürlichen oder synthetischen, nicht polymeren Verdickungsmitteln, Suspensionsmitteln, Komplexbildnern, Reduktionsmitteln, Trübungsmitteln, Farbstoffen, Sonnenschutzfiltern, Vitaminen oder Provitaminen, Duftstoffen und Konservierungsstoffen, Mitteln zur Einstellung des pH-Werts, Konditionierungsmitteln und ihren Mischungen.

11. Verwendung nach einem der vorhergehenden Ansprüche, **dadurch gekennzeichnet, dass** sie eine oder mehrere organische Säuren umfasst.

12. Verwendung nach Anspruch 11, **dadurch gekennzeichnet, dass** die organische Säure oder die organischen Säuren aus Ethansäure, Propansäure, Butansäure, Milchsäure, Glycolsäure, Ascorbinsäure, Maleinsäure, Phthalsäure, Bernsteinsäure, Taurin, Weinsäure, Gluconsäure, Glucuronsäure und Zitronensäure ausgewählt ist bzw. sind.

13. Haarkosmetische Zusammensetzung, die mindestens ein Silikon und mindestens ein Alkoxysilan umfasst, das mindestens eine löslichkeitserhöhende funktionelle Gruppe enthält, nach Anspruch 1, wobei das oder die Silikon(e) aus organomodifizierten Polysiloxanen ausgewählt ist bzw. sind, die aus Polydialkylsiloxanen, vorzugsweise Polydimethylsiloxanen ausgewählt sind, organomodifiziert mit Aminogruppen, C₁-C₄-Alkoxygruppen, Carboxylgruppen, Acrylgruppen, Polyaminogruppen.

## Claims

1. Use of at least one silicone in a cosmetic composition for shaping the hair comprising at least one alkoxysilane comprising at least one solubilizing functional group chosen from primary, secondary and tertiary amine, aromatic amine, alcohol, carboxylic acid, sulfonic acid, anhydride, carbamate, urea, guanidine, aldehyde, ester, amide, epoxy, pyrrole, dihydroimidazole, gluconamide, pyridyl and polyether groups, in order to avoid damaging the feel of the hair during the repeated application of the said composition for shaping the hair;
the alkoxysilane or alkoxysilanes comprising at least one solubilizing functional group are chosen from the compounds of formula: 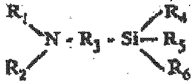 in which:
R₄ represents a halogen or an OR' or R'₁ group;
R₅ represents a halogen or an OR" or R'₂ group;
R₆ represents a halogen or an OR"' or R'₃ group;
R₁, R₂, R₃, R', R", R"', R'₁, R'₂ and R'₃ represent, independently of one another, a saturated or unsaturated and linear or branched hydrocarbon group optionally carrying additional chemical groups, it being possible for R₁, R₂, R', R" and R'" to additionally denote hydrogen, at least two of the R₄, R₅ and R₆ groups being different from the R'₁, R'₂ and R'₃ groups and at least two of the R', R" and R"' groups being different from hydrogen;
the silicone or silicones being chosen from organomodified polysiloxanes chosen from polydialkylsiloxanes, preferably polydimethylsiloxanes, organomodified by amine, C₁-C₄ alkoxy, carboxyl, acrylic or polyamine groups.

2. Use according to Claim 1, **characterized in that** the R₁, R₂, R', R'₁, R'₂, R'₃, R" and R"' groups are chosen from C₁-C₁₂ alkyl, C₆ to C₁₄ aryl, C₁ to C₈ alkyl-C₆ to C₁₄ aryl and C₆ to C₁₄ aryl-C₁ to C₈ alkyl radicals.

3. Use according to Claim 1, **characterized in that** the alkoxysilane or alkoxysilanes comprising at least one solubilizing functional group are chosen from the compounds of formula: 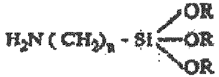 in which the R radicals, which are identical or different, are chosen from C₁-C₆ alkyl radicals and n is an integer from 1 to 6, preferably from 2 to 4.

4. Use according to Claim 3, **characterized in that** the alkoxysilane comprising at least one solubilizing functional group is (γ-aminopropyl)triethoxysilane.

5. Use according to any one of the preceding claims, **characterized in that** the alkoxysilane or alkoxysilanes comprising at least one solubilizing functional group represent between 0.1 and 20% by weight, preferably between 1 and 15% by weight, of the total weight of the composition.

6. Use according to any one of the preceding claims, **characterized in that** the silicone or silicones represent between 0.01 and 20% by weight, preferably between 0.1 and 10% by weight and better still between 0.1 and 5% by weight of the total weight of the composition.

7. Use according to any one of the preceding claims, **characterized in that** the composition comprises at least one solvent chosen from water, C₁-C₈ alcoholic solvents and their mixtures.

8. Use according to Claim 7, **characterized in that** the C₁-C₈ alcoholic solvents are chosen from alkanols, alkanediols, benzyl alcohol and phenylethyl alcohol.

9. Use according to Claim 8, **characterized in that** the solvents are chosen from ethanol, propanol or isopropanol.

10. Use according to any one of the preceding claims, **characterized in that** it additionally comprises at least one cosmetic active principle chosen from protein hydrolysates, swelling and penetrating agents, agents for combating hair loss, anti-dandruff agents, natural or synthetic non-polymeric thickeners, suspending agents, sequestering agents, reducing agents, opacifying agents, colourants, sunscreens, vitamins or provitamins, fragrances and preservatives, pH adjusters, conditioning agents and their mixtures.

11. Use according to any one of the preceding claims, **characterized in that** it comprises one or more organic acids.

12. Use according to Claim 11, **characterized in that** the organic acid or acids are chosen from acetic acid, propanoic acid, butanoic acid, lactic acid, glycolic acid, ascorbic acid, maleic acid, phthalic acid, succinic acid, taurine, tartaric acid, gluconic acid, glucuronic acid and citric acid.

13. Hair cosmetic composition comprising at least one silicone and at least one alkoxysilane comprising at least one solubilizing functional group as defined in Claim 1, the silicone or silicones being chosen from organomodified polysiloxanes chosen from polydialkylsiloxanes, preferably polydimethylsiloxanes, organomodified by amine, C₁-C₄ alkoxy, carboxyl, acrylic or polyamine groups.
